## Europäisches Patentamt

## European Patent Office

## Office européen des brevets

(19)

(11) Veröffentlichungsnummer: **0 207 344**
**B1**

(12) **EUROPÄISCHE PATENTSCHRIFT**

(45) Veröffentlichungstag der Patentschrift:
**01.02.89**

(51) Int. Cl.⁴: **A23K 1/16**

(21) Anmeldenummer: **86108094.3**

(22) Anmeldetag: **13.06.86**

(54) Verfahren zur Herstellung staubfreier Olaquindox-haltiger Futtermittelmischungen.

(30) Priorität: **27.06.85 DE 3522937**

(43) Veröffentlichungstag der Anmeldung:
**07.01.87 Patentblatt 87/2**

(45) Bekanntmachung des Hinweises auf die Patenterteilung:
**01.02.89 Patentblatt 89/5**

(84) Benannte Vertragsstaaten:
**AT BE CH DE FR GB IT LI NL SE**

(56) Entgegenhaltungen:
**EP-A- 0 134 444**
**DE-A- 2 541 459**

(73) Patentinhaber: **BAYER AG, Konzernverwaltung RP Patentabteilung, D-5090 Leverkusen 1 Bayerwerk(DE)**

(72) Erfinder: **Voege, Herbert, Dr., Martin-Buber-Strasse 41, D-5090 Leverkusen 3(DE)**
Erfinder: **Sieveking, Hans-Ulrich, Dr., Walter-Flex-Strasse 23, D-5090 Leverkusen(DE)**

ACTORUM AG

## Beschreibung

Die vorliegende Erfindung betrifft ein Verfahren zur Herstellung staubfreier Olaquindox-haltiger Futtermittelvormischungen sowie Futtermittelvormischungen die nach diesem Verfahren hergestellt sind.

Olaquindox (2-[N-(2-Hydroxyethyl)-carbamoyl]-3-methyl-chinoxalin-1,4-dioxid) wird als Wirkstoff in Tierfuttermittelmischungen eingesetzt. Es sind Schwierigkeiten bei der Herstellung staubfreier Olaquindox-haltiger Tierfuttermittelmischungen aufgetreten.

Es ist bekannt, die Staubentwicklung bei der Herstellung einer wirkstoffhaltigen Mischung dadurch zu reduzieren, daß auf Trägermaterialien eine Wirkstofflösung oder Suspension aufgebracht und diese gegebenenfalls unter Zusatz von Polymeren auf dem Trägerstoff fixiert wird.

Ebenso ist es bekannt, organische Trägermaterialien zunächst mit Öl zu mischen, Wirkstoffe wie z.B. Chinoxalin-1,4-dioxide zuzugeben und diesem Gemisch weiteres Öl zuzumischen (DE-OS 2 541 459).

Die vorliegende Erfindung betrifft ein Verfahren zur Herstellung Olaquindox-haltiger staubfreier Tierfuttermittelvormischungen das dadurch gekennzeichnet ist, daß man

a) 35 bis 98,5 Gew.-% eines für die Ernährung von Tieren zugelassenen anorganischen Trägermaterials mit einem Maximum der Korngrößenverteilung zwischen 0,1–1,0 mm mit

b) 0,5–10 Gew.-% eines nichttoxischen Öles und/oder einem zur Tierernährung zugelassenen Emulgator mischt und dieser Mischung

c) 1–55 Gew.-% Olaquindox und gegebenenfalls weitere Hilfsstoffe zugibt.

Besonders bevorzugt ist ein Verfahren zur Herstellung Olaquindox-haltiger staubfreier Tierfuttermittelvormischungen dadurch gekennzeichnet, daß man

a) 75 bis 98,5 Gew.-% eines für die Ernährung von Tieren zugelassenen anorganischen Trägermaterials mit einem Maximum der Korngrößenverteilung zwischen 0,1 bis 10 mm mit

b) 0,5–5 Gew.-% eines nichttoxischen Öles und/oder einem zur Tierernährung zugelassenen Emulgator mischt und dieser Mischung

c) 1–20 Gew.-% Olaquindox und gegebenenfalls weitere Hilfsstoffe zugibt.

Organische Trägermaterialien wie sie nach dem Stand der Technik für die Herstellung staubfreier Futtermittelvormischungen eingesetzt werden haben den Nachteil, daß sie relativ teuer sind. Außerdem bereiten sie bei der Lagerung dadurch Probleme, daß sie gegen Schädlingsbefall geschützt werden müssen. Auch ist es aufwendig, sie in einheitlicher Korngrößenverteilung herzustellen.

Alle diese Nachteile können beim Einsatz anorganischer Trägermaterialien umgangen werden. Allerdings bereitet das Aufbringen des Wirkstoffs Olaquindox auf die anorganischen Trägermaterialien mit Hilfe der als Futterzusatzmittel zugelassenen Stoffe Schwierigkeiten. Öle benetzen den anorganischen Träger nur schlecht. Sie bilden auf seiner Oberfläche kleine Tröpfchen. Dies führt zu ungleichmäßigem Auftrag des Wirkstoffs sowie zu Verklumpung der Mischung nach Beendigung des Mischvorgangs.

Durch den Einsatz einer Mischung aus Öl und Emulgator oder durch Arbeiten mit Emulgator allein können diese Schwierigkeiten überwunden werden.

Als Trägermaterialien kommen alle anorganischen Materialien, die in der Tierernährung eingesetzt werden können, in Betracht (siehe z.B. Futtermittelverordnung, Teil 1.3 Mineral Einzelfuttermittel sowie Teil 2 bzw. EG Richtlinien 77/101 EWG, Anhang B4). Dies sind bevorzugt, aber nicht ausschließlich, Futterkalk (Calciumcarbonat), Kochsalz oder Calciumphosphate. Diese Trägerstoffe dürfen eine bestimmte Korngröße nicht über- oder unterschreiten. Bei Einsatz von Pulver backen die Partikel zusammen. Bei zu grobkörnigem Material wird die Verteilung des Wirkstoffes im Endfutter inhomogen und Entmischungen können stattfinden. Die Trägerstoffe müssen daher ein Korngrößen-Verteilungs-Maximum zwischen 0,1 und 1 mm haben.

Als Öle kommen alle für die Anwendung in Futtermitteln zugelassenen und geeigenten Öle, besonders pflanzlicher Herkunft, in Frage. Aber auch andere Öle, z.B. Paraffinöle, können eingesetzt werden.

Als Emulgatoren kommen alle diejenigen in Betracht, die in Futtermitteln eingesetzt werden können. (Richtlinie 70/524 EWG, Anhang I, Abschn. E, Anhang II, Abschn. C).

Es sind dies z.B. höhere Alkylester der Essigsäure, Milchsäure, Weinsäure, Citronensäure, Monoacetyl- und Diacetyl-Weinsäure, Mono- und Diglyceride von Speisefettsäuren,

Ester von Zucker wie z.B. Saccharose mit Speisefettsäuren, Glycerinpolyethylenglykolricinoleat, Monoester von Speisefettsäuren mit 1,2 Propylenglykol oder Polyethylenglykolen.

Andere Hilfsstoffe sind z.B. Antioxydantien wie Butylhydroxytoluol, Ascorbylpalmitat, Tocopherol oder Ethoxyquin, ferner Aroma- und Geschmacksstoffe oder Farbstoffe.

Vorzugsweise wird bei Olaquindox- Futtermittelvormischungen der Wirkstoff zwischen 1% und 20%, vorzugsweise zwischen 2% und 15% eingesetzt. Entsprechend liegt der Anteil des Trägerstoffes zwischen 75% und 98,5%, vorzugsweise zwischen 80% und 97,5%.

Als Trägerstoffe kommen bevorzugt Calciumcarbonat, Natriumchlorid und sek. Calciumphosphat in Frage.

Das Korngrößen-Verteilungs-Maximum der eingesetzten Trägerstoffe liegt zwischen 0,1 mm und 1,0 mm, vorzugsweise zwischen 0,1 mm und 0,8 mm.

Der Anteil des Öles und/oder Emulgator kann je nach Art des Trägers zwischen 0,5 und 10 Gew.-% variieren, vorzugsweise zwischen 1 und 5 Gew.-% und insbesondere zwischen 1 und 3 Gew.-%.

Als Öle kommen vorzugsweise Öle pflanzlichen Ursprungs, wie Erdnußöl, Sonnenblumenöl oder Sojaöl in Betracht. Bei den Emulgatoren finden vorzugsweise Mono- und Diglyceride von Speisefettsäuren, Glycerinpolyethylenglykolricinoeat, Propylenglykol-Monoester, Polyethylenglykolester von Speisefettsäuren sowie Zuckerester in Betracht.

Zur Herstellung der beanspruchten Formulierungen wird der Trägerstoff in einem herkömmlichen Mischer vorgelegt. In den laufenden Mischer wird die Öl- und/oder Emulgator-Lösung gegeben – evtl. mit weiteren Hilfsstoffen – und so lange gemischt, bis das Trägermaterial gleichmäßig benetzt ist. Die Mischzeit ist von der Art des Mischers abhängig. Dann wird der Wirkstoff zugegeben und weiter gemischt, bis eine homogene Mischung entstanden ist.

Die folgenden Beispiele sollen die beanspruchten Formulierungen verdeutlichen, aber nicht eingrenzen.

Die Herstellung der Vormischungen erfolgt auf die oben angeführte Weise. Die Werte hinter dem Träger geben das Maximum der Kornverteilung an.

### Beispiel 1

| | |
|---|---|
| Natriumchlorid 0,2 mm | 88,0 g |
| Mono-Diglyceridgemisch von Fettsäuren | 2,0 g |
| Olaquindox | 10,0 g |
| | 100,0 g |

### Beispiel 2

| | |
|---|---|
| Calciumcarbonat | 97,0 g |
| Erdnussöl mit 0,1% Antioxydans | 0,5 g |
| Glycerinpolyethylenglykolricinoleat | 0,5 g |
| Olaquindox | 2,0 g |
| | 100,0 g |

Öl und Emulgator werden vor Einsatz gemischt.

### Beispiel 3

| | |
|---|---|
| sek. Calciumphosphat 0,4 mm | 85,0 g |
| Glycerinpolyethylenglykolricinoleat | 5,0 g |
| Olaquindox | 10,0 g |
| | 100,0 g |

### Beispiel 4

| | |
|---|---|
| Calciumcarbonat 0,2 mm | 85,0 g |
| Sonnenblumenöl + 0,1% Antioxydans | 1,5 g |
| Monoglycerid-Fettsäureester | 0,5 g |
| Olaquindox | 5,0 g |
| | 100,0 g |

Öl und Emulgator werden vor Einsatz gemischt.

### Beispiel 5

| | |
|---|---|
| Calciumcarbonat 0,2 mm | 88,5 g |
| Glycerinpolyethylenglykolricinoleat | 1,5 g |
| Olaquindox | 10,0 g |
| | 100,0 g |

### Beispiel 6

| | |
|---|---|
| Calciumcarbonat 0,2 mm | 44,0 g |
| Glycerinpolyethylenglykolricinoleat | 6,0 g |
| Olaquindox | 50,0 g |
| | 100,0 g |

### Beispiel 7

| | |
|---|---|
| sek. Calciumphosphat 0,4 mm | 69,0 g |
| Glycerinpolyethylenglykolricinoleat | 6,0 g |
| Olaquindox | 25,0 g |
| | 100,0 g |

### Beispiel 8

| | |
|---|---|
| Calciumcarbonat | 55,0 g |
| Erdnussöl mit 0,1% Antioxydans | 2,5 g |
| Glycerinpolyethylenglykolricinoleat | 2,5 g |
| Olaquindox | 40,0 g |
| | 100,0 g |

Die Formulierungen nach obigen Beispielen wurden vergleichend gegen herkömmliche Olaquindox-Futtermittelvormischungen einem Staubtest unterworfen. Dabei wird in einer handelsüblichen Testapparatur Staub erzeugt. Durch die Apparatur wird unter definierten Bedingungen Luft gesaugt und der Staub auf einem Membranfilter aufgefangen. Das Filter wird mittels der HPLC-Analysenmethode auf Wirkstoff-Abscheidung geprüft. Einzelheiten des Tests siehe D. Stauber und R. Beutel, Fresenius Z. Anal. Chem. (1984) 318, 522–524.

Während bei den herkömmlichen Olaquindox-Futtermittelvormischungen Olaquindox Staub nachgewiesen werden konnte, war dies bei Formulierungen nach obigen Beispielen nicht der Fall.

## Patentansprüche

1. Verfahren zur Herstellung Olaquindox-haltiger staubfreier Tierfuttermittelvormischungen dadurch gekennzeichnet, daß man
a) 35 bis 98,5 Gew.-% eines für die Ernährung von Tieren zugelassenen anorganischen Trägermaterials mit einem Maximum der Korngrößenverteilung zwischen 0,1–1,0 mm mit
b) 0,5–10 Gew.-% eines nichttoxischen Öles und/oder einem zur Tierernährung zugelassenen Emulgator mischt und dieser Mischung
c) 1–55 Gew.-% Olaquindox und gegebenenfalls weitere Hilfsstoffe zugibt.

2. Verfahren gemäß Anspruch 1, dadurch gekennzeichnet, daß man
a) 75 bis 98,5 Gew.-% eines für die Ernährung von Tieren zugelassenen anorganischen Trägermaterials mit einem Maximum der Korngrößenverteilung zwischen 0,1–1,0 mm mit
b) 0,5–5 Gew.-% eines nichttoxischen Öles und/oder einem zur Tierernährung zugelassenen Emulgator mischt und dieser Mischung

c) 1–20 Gew.-% Olaquindox zugibt.

3. Verfahren gemäß Anspruch 1 und 2, dadurch gekennzeichnet, daß als Emulgatoren höhere Alkylester von Milchsäure, Essigsäure, Weinsäure und Zitronensäure, Mono- und Diglyceride mit Speisefettsäuren, Glycerinpolyethylenglykolricinoleat, 1,2-Propylenglykolmonoester oder Polyethylenglykolester von Speisefettsäuren sowie Ester von Zucker mit Speisefettsäuren eingesetzt werden.

4. Verfahren gemäß Anspruch 1 und 2, dadurch gekennzeichnet, daß als anorganische Träger Calciumcarbonat, Calciumphosphat oder Kochsalz eingesetzt werden.

## Revendications

1. Procédé pour la production de prémélanges d'aliments pour bétail exempts de poussière et contenant de l'Olaquindox, caractérisé en ce que l'on mélange

a) 35 à 98,5 en poids d'un matériau de support inorganique autorisé pour l'alimentation des animaux et avec un maximum de répartition granulométrique compris entre 0,1 et 1,0 mm à

b) 0,5 à 10% en poids d'une huile non toxique et/ou d'un émulsifiant autorisé pour l'alimentation des animaux et que l'on ajoute à ce mélange

c) 1–55% en poids d'Olaquindox et éventuellement d'autres adjuvants.

2. Procédé selon la revendication 1, caractérisé en ce que l'on mélange

a) 75 à 98,5% en poids d'un matériau de support inorganique autorisé pour alimentation des animaux et avec un maximum de répartition granulométrique compris entre 0,1 et 1,0 mm à

b) 0,5 à 5% en poids d'une huile non toxique et/ou d'un émulsifiant autorisé pour l'alimentation des animaux et que l'on ajoute à ce mélange.

c) 1 à 20% en poids d'Olaquindox.

3. Procédé selon les revendications 1 et 2, caractérisé en ce que l'on utilise comme émulsifiant, des esters alkyliques supérieurs, de l'acide lactique, de l'acide acétique, de l'acide tartrique et de l'acide citrique, des mono- et diglycérides d'acides gras alimentaires, du ricinoléate de glycérine polyethylène glycol, des mono-esters 1,2 propylène glycoliques ou des esters polyethylène glycoliques d'acides gras alimentaires, ainsi que des esters des sucres avec des acides gras alimentaires.

4. Procédé selon les revendications 1 et 2, caractérisé en ce que l'on utilise comme support inorganique du carbonate de calcium, du phosphate de calcium ou du sel de cuisine.

## Claims

1. Process for the production of olaquindox-containing dust-free livestock feedstuff premixes, characterized in that

a) 35 to 98.5% by weight of an inorganic carrier material which is approved for livestock nutrition and has a maximum particle of the size distribution between 0.1–1.0 mm is mixed with

b) 0.5–10% by weight of a non-toxic oil and/or with an emulsifier approved for livestock nutrition, and to this mixture is added

c) 1–55% by weight of olaquindox and, where appropriate, other auxiliaries.

2. Process according to Claim 1, characterized in that

a) 75 to 98.5% by weight of an inorganic carrier material which is approved for livestock nutrition and has a maximum of the particle size distribution between 0.1–1.0 mm is mixed with

b) 0.5–5% by weight of a non-toxic oil and/or with an emulsifier approved for livestock nutrition, and to this mixture is added

c) 1–20% by weight of olaquindox.

3. Process according to Claim 1 and 2, characterized in that higher alkyl esters of lactic acid, acetic acid, tartaric acid and citric acid, mono- and diglycerides with edible fatty acids, glycerol polyethylene glycol ricinoleate, 1,2-propylene glycol monoesters or polyethylene glycol esters of edible fatty acids, and esters of sugar with edible fatty acids are used as emulsifiers.

4. Process according to Claim 1 and 2, characterized in that calcium carbonate, calcium phosphate or sodium chloride are used as inorganic carriers.